# EUROPEAN PATENT APPLICATION

(11) **EP 2 223 923 A1**
(43) Date of publication of application: **01.09.2010**
(21) Application number: 09382024.9
(22) Date of filing: 25.02.2009
(51) Int. Cl.: C07D 487/04

(54) **Process for the preparation of a chiral beta aminoacid derivative and intermediates thereof**

(71) Applicant: Esteve Química, S.A., 08024 Barcelona (ES)
(72) Inventor: Bartra Sanmarti, Martí, 08024, Barcelona (ES); Rustullet Oliver, Albert, 08024, Barcelona (ES); Fernandez Hernandez, Sara, 08024, Barcelona (ES); Monsalvatje Llagostera, Montserrat, 08024, Barcelona (ES)
(74) Representative: ZBM Patents

(57) **Abstract**

It comprises a process for the preparation of sitagliptin, or its pharmaceutically acceptable salts, or its solvates, including hydrates, comprising: a) coupling an halo-2,4,5-trifluorobenzene with a compound of formula (IV) to give N-protected sitagliptin; the coupling being carried out via the formation of an organocupric compound of the halo-2,4,5-trifluorobenzene or, alternatively, via the formation of a organozinc compound of a compound of formula (IV); where R₁ is hydrogen or an amino protective group; R₂ is an amino protective group; or alternatively R₁ and R₂ taken together form a phtalimido group; X is Br or I; and Y is r, I or R₃SO₂- wherein R₃ is (C₁-C₄)-alkyl, phenyl, or phenyl mono- or disubstituted by a (C₁-C₄)-alkyl radical; b) submitting the N-protected sitagliptin to a deprotection reaction; and c) optionally its conversion into a pharmaceutically acceptable salt. It also comprises new intermediate compounds useful in such preparation process

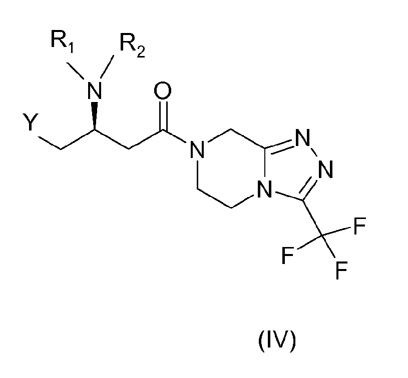

## Description

The present invention relates to a process for the preparation of sitagliptin, as well as to some new intermediates useful in such preparation process.

### BACKGROUND ART

Sitagliptin is the International Non-proprietary Name (INN) of (*R*)-3-amino-1-[3-(trifluoromethyl)-5,6-dihydro-[1,2,4]triazolo[4,3-*a*]pyrazin-7(8*H*)-yl]-4-(2,4,5-trifluorophenyl)butan-1-one, and CAS No. 486460-32-6. Sitagliptin is an oral antihyperglycemic of the dipeptidyl peptidase-4 inhibitor class. Sitagliptin phosphate monohydrate (CAS No. 65471-77-9) is currently used either alone or in combination with other oral antihyperglycemic agents for treatment of diabetes mellitus type 2.

The structure of sitagliptin phosphate monohydrate corresponds to the following formula:

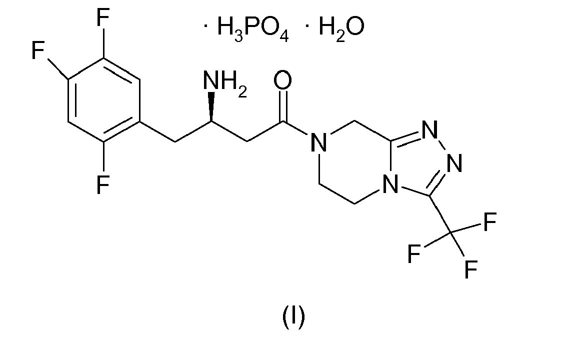

Different synthetic strategies for the preparation of sitagliptin and its salts are known.

WO 2003004498 discloses a class of beta-amino tetrahydrotriazolo[4,3-α]pyrazines, including sitagliptin, methods for their preparation, and pharmaceutical compositions using these compounds. A preparation process of sitagliptin hydrochloride is reported in Example 7 of this document. According to this Example, preparation of sitaglipin hydrochloride proceeds through the coupling of (3R)-3-[(1,1-dimethylethoxycarbonyl)-amino]-4-(2,4,5-trifluorophenyl)butanoic acid and 3-(trifluoromethyl)-5,6,7,8-tetrahydro-1,2,4-triazolo[4,3-α]pyrazine under standard peptide coupling conditions, followed by the removal of the protecting group with methanolic hydrogen chloride.

Other processes are based on carrying out an asymmetric hydrogenation of the corresponding prochiral beta amino acrylic acid derivative. Thus, WO 2004085378 describes a process for the preparation of enantiomerically enriched beta chiral amino acid derivatives, including sitagliptin, based on carrying out a transition metal-catalyzed asymmetric hydrogenation of the corresponding prochiral beta amino acrylic acid derivative having the primary amino group unprotected, in the presence of a transition metal precursor complexed with a chiral ferrocenyl diphosphine ligand. In particular, in Example 1 sitagliptin is obtained by a process comprising the following steps:
(a) coupling between 3-(trifluoromethyl)-5,6,7,8-tetrahydro[1,2,4]triazolo[4,3-a]pyrazine and the adduct obtained by reaction of 2-(trifluorophenyl)acetic acid with Meldrum's acid by means of pivaloyl chloride and diethylamine in dimethylacetamide, to provide the corresponding 3-oxobutyramide derivative;
(b) reacting the compound obtained with ammonium acetate and ammonium hydroxide to give the corresponding prochiral beta amino acrylic acid derivative; (c) submitting the compound obtained to a asymmetric hydrogenation in the presence of chloro(1,5-cyclooctadiene)rhodium (I) dimmer and (R,S) tert-butyl Josiphos as chiral catalyst, to yield the sitagliptin.

In WO 2005097733 the asymmetric hydrogenation is carried out in the presence of a rhodium metal precursor complexed with a chiral mono- or biphosphine ligand.

Finally, in WO 2006081151 the asymmetric hydrogenation is carried out in the presence of an ammonium salt and a transition metal precursor complexed with a chiral ferrocenyl diphosphine ligand.

Unfortunately, these processes based on carrying out an asymmetric hydrogenation suffer from operation difficulty due to the high hydrogen pressures of about 100-500 psi needed to carry out the reaction. That means that special facilities which tolerate the work at high pressures are required. Another drawback of the asymmetric hydrogenation is the very high cost of the catalyst and its ligand. Although Josiphos ligands are commercial, their cost is still very high.

WO 2004085661 discloses a process for the preparation of enantiomerically-enriched beta-amino acid derivatives involving the elaboration of pure Z-enamines from beta-ketoesters and amides using (S)-phenylglycine amide, and their subsequent hydrogenation using heterogeneous catalysis. Hydrogenolytic cleavage of the (S)-phenylglycine amide obtained affords the corresponding enantiomerically enriched beta-amino acid derivatives.

WO 2004087650 discloses a process for the preparation of sitagliptin, among other chiral beta aminoacid derivatives, based on submitting the compound 7-[(3R)-3-[(benzyloxy)amino]-4-(2,4,5-trifluorophenyl)butanoyl]-3-(trifluoromethyl)-5,6,7,8-tetrahydro[1,2,4]triazolo[4,3-a]pyrazine to an hydrogenation reaction with H₂ over Pd/C in methanol. The starting material is obtained by coupling (3R)-3-[(benzyloxy)amino]-4-(2,4,5-trifluorophenyl)butanoic acid and 3-(trifluoromethyl)-5,6,7,8-tetrahydro-1,2,4-triazolo[4,3-α]pyrazine.

Despite the teaching of all these prior art documents, the research of new preparation processes of sitagliptin is still an active field, since the industrial exploitation of known processes is difficult, as it has been pointed out in many of the above-cited documents. Thus, the provision of new preparation processes of sitagliptin is desirable.

### SUMMARY OF THE INVENTION

Inventors have found a new process for the preparation of sitagliptin which avoids the main disadvantages of the known processes. Among the striking advantages of this preparation process the following can be mentioned: it avoids working with catalysts and ligands of high cost as the rhodium and the Josiphos ligands; it also avoids working at high pressures of hydrogen which is advantageous from the point of view of the process implementation; and the starting material, (S)-benzyl 5-oxotetrahydrofuran-3-ylcarbamate, contains the chiral center of the final compound, which avoids the need to carry out a estereoselective reaction in the last step and, therefore, the risk of e.e. lost when scale up the reaction.

Thus, an aspect of the present invention is the provision of a process for the preparation of sitagliptin of formula (I), or its pharmaceutically acceptable salts, or its solvates, including hydrates, 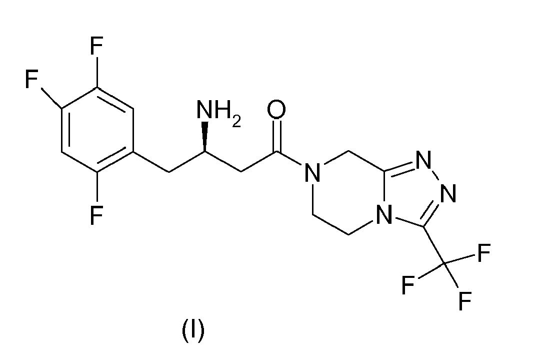 which comprises the steps of: a) coupling a compound of formula (III) with a compound of formula (IV) to give a compound of formula (II); 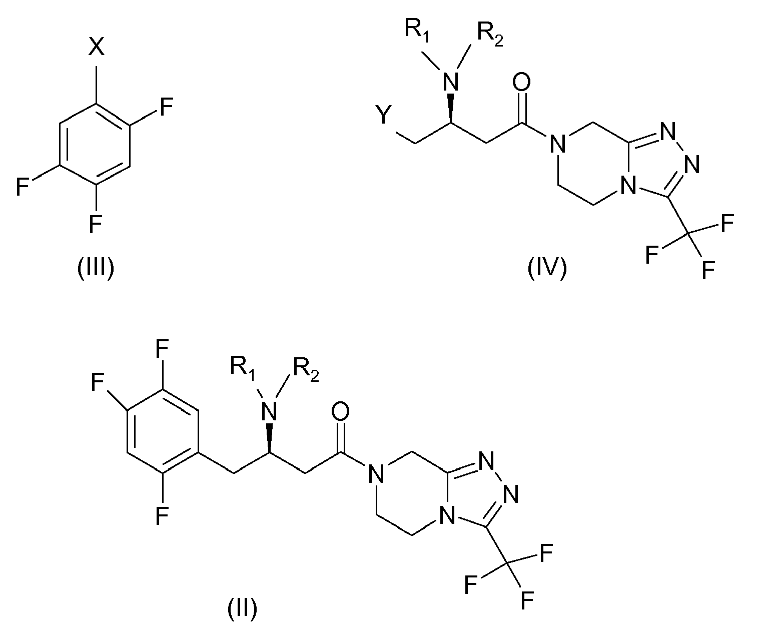 the coupling being carried out first via the formation of an organocupric compound of the compound of formula (III) by reacting the compound (III) with magnesium or lithium to give an organomagnesium or an organolithium compound, and then with a copper salt to give an organocupric compound or, alternatively, first via the formation of a organozinc compound of the compound of formula (IV) by reacting compound (IV) with zinc, and then carrying out the coupling of the organozinc compound thus obtained with the compound of formula (III) in the presence of a palladium catalyst, where in the previous formulae R₁ is an hydrogen atom or an amino protective group; R₂ is an amino protective group; or, alternatively, R₁ and R₂ taken together form a phtalimido group; X is Br or I; and Y is selected from Br, I, and a radical of formula R₃SO₂-, where R₃ is a radical selected from (C₁-C₄)-alkyl, phenyl, and phenyl mono- or disubstituted by a (C₁-C₄)-alkyl radical; b) submitting the compound (II) obtained in step a) to a deprotection reaction to remove the protective groups to yield sitagliptin (I) as free base; and c) optionally, treating the compound obtained in step b) with a pharmaceutically acceptable acid to form the corresponding pharmaceutically acceptable salt.

Compound of formula (IV) where Y is selected from Cl, Br, I and a radical of formula R₃SO₂-, where R₃ is a radical selected from (C₁-C₄)-alkyl, phenyl, and phenyl mono- or disubstituted by a (C₁-C₄)-alkyl radical; can be obtained from compound of formula (V) where R₁ and R₂ have the same meanings as in compound (IV) by several methods explained in detail below.

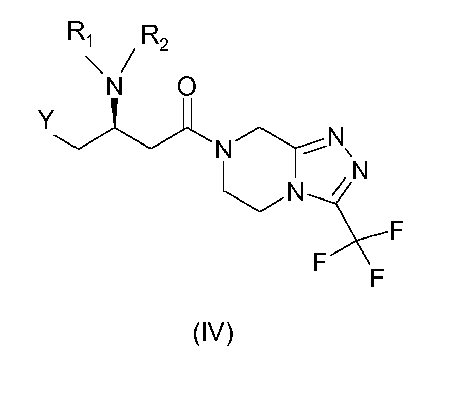

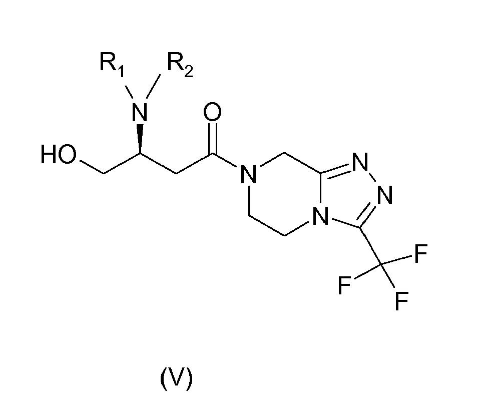

Intermediate compounds of formula (IV) where R₁ is an hydrogen atom or an amino protective group; R₂ is an amino protective group; or, alternatively, R₁ and R₂ taken together form a phtalimido group; Y is selected from Cl, Br, I, and a radical of formula R₃SO₂-, where R₃ is a radical selected from (C₁-C₄)-alkyl, phenyl, and phenyl mono- or disubstituted by a (C₁-C₄)-alkyl radical with the proviso that when R₁ is H and R₂ is t-butoxycarbonyl, then R₃ is not methyl, are new. Intermediate compounds of formula (V) where R₁ is a hydrogen atom or an amino protective group; R₂ is an amino protective group; or, alternatively, R₁ and R₂ taken together form a phtalimido group with the proviso that when R₁ is H then R₂ is not t-butoxycarbonyl, are also new. Thus, the provision of these new compounds of formulae (IV) and (V), which are useful intermediates for the preparation of sitagliptin, also form part of the present invention. These intermediates allow the preparation of sitagliptin by a simple process which proceeds with high yields and purity.

### DETAILED DESCRIPTION OF THE INVENTION

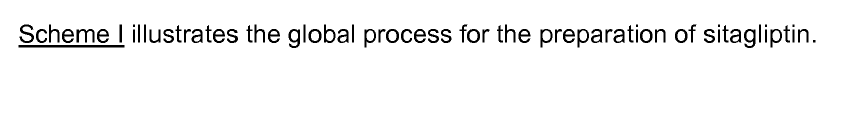

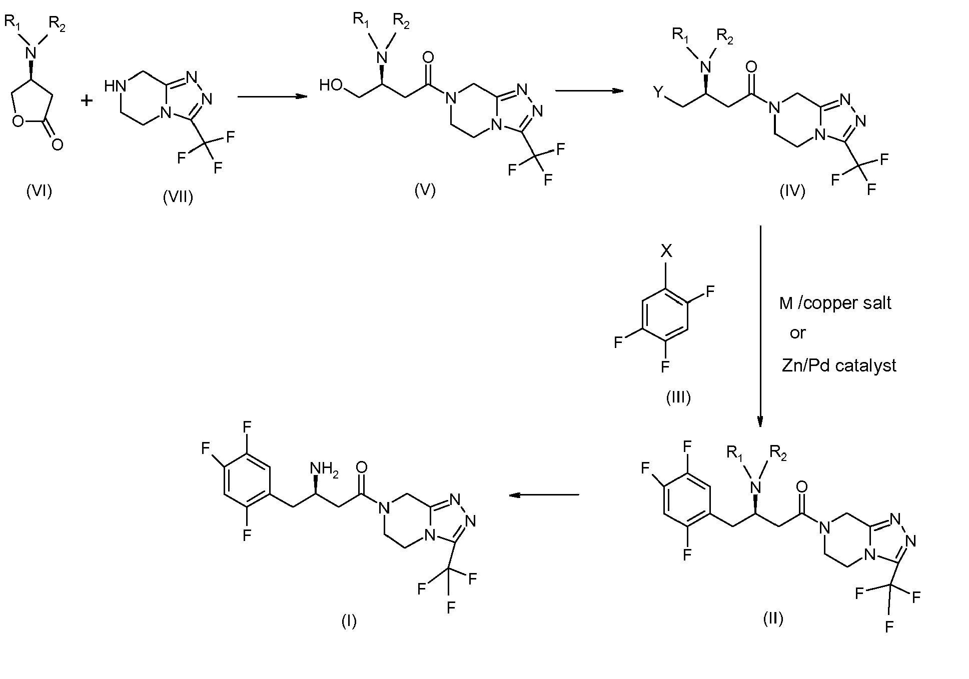

In the previous Scheme M is a metal selected from Mg or Li, and R₁, R₂, X, and Y have the meaning mentioned above.

It is part of the present invention the provision of single reaction steps of the global process of Scheme I to obtain sitagliptin, as well as the combination of two or more sequential steps of the global process.

As it is illustrated in the previous Scheme I, compound of formula (V) can be prepared by reaction of compound (VI) where R₁ is a hydrogen atom or an amino protective group; R₂ is an amino protective group; or, alternatively, R₁ and R₂ taken together form a phtalimido group, with compound (VII).

In a preferred embodiment, R₁ is hydrogen and R₂ is the amino protective group.

Suitable amino protective groups for the amino moiety are selected from those known in the art. Preferably, the amino protective group is selected from carbamates, amides, sulfonamides, and benzylamines. In a preferred embodiment, the amino protective group is selected from *t*-butoxycarbonyl (BOC), 9-fluorenylmethoxycarbonyl (Fmoc), benzyloxycarbonyl (Cbz), and N-benzylamine. In a more preferred embodiment the amino protective group is the benzyloxycarbonyl.

The amino protective group is maintained in the subsequent reaction steps up to the end of the process, being the last step a deprotection reaction to remove the protective group.

Compound (VI) is also known in the literature and may be conveniently prepared by a variety of methods known to those skilled in the art (cf. e.g. Org. Biomolec. Chem. 2004, vol. 2, p. 2003).

Compound (VII) is also known in the literature and may be conveniently prepared by a variety of methods known to those skilled in the art (cf. e.g. EP 1412357, or Drugs of the Future 2005, vol. 30 No. 4, pp. 337-343). Compound (VII) in form of hydrochloride is commercially available and can be easily converted into its free base by reaction with sodium carbonate in methanol as solvent.

The reaction between compound (VI) and (VII) can be carried out without solvent or in the presence of an appropriate solvent. Appropriate solvents include for instance (C₁-C₃)-chlorine containing solvents such as dichloromethane, (C₆-C₈)-aromatic hydrocarbons such as toluene or xylene, (C₄-C₆)-alcohols such as n-butanol, tertiary amines such as N-methyl morpholine and mixtures of solvents. Optionally, the reaction can be carried out in the presence of a Lewis acid as catalyst. Examples of appropriate Lewis acid are AlMe₃, AlCl₃, TiCl₄, ZrCl₄, or FeCl₃- In a preferred embodiment, the Lewis acid is AlMe₃ or AlCl_{3.}In a more preferred embodiment, the catalyst is AlMe₃. In a particular embodiment, the catalyst is AlCl₃. When AlCl₃ is used as catalyst, triethylamine can be used as additive.

The conversion of compound (V) to compound (IV) can be carried out by reacting a compound of formula (V) with an appropriate reagent system to convert the OH functional group to a leaving group Y, yielding the compound of formula (IV); and thereafter as necessary transforming the compound of formula (IV) obtained into another compound of formula (IV) as defined above.

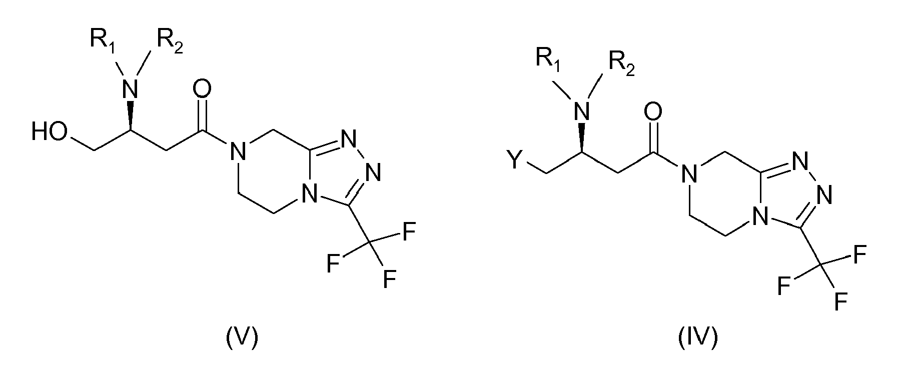

In the previous formulae, R₁, R₂ and Y have the same meaning as defined above, Y including Cl.

The conversion of compound (V) to compound (IV) can be carried out with reagents that convert directly the OH functional group to a leaving group, or through the activation of the compound (V).

In a particular embodiment of the process, compounds of formula (V) are those where R₁ is H and R₂ is an amino protective group as defined above. In a particular embodiment of the process, compounds of formula (V) are those
where R₁ is hydrogen and R₂ is benzyloxycarbonyl.

In a preferred embodiment of this step of the process, the appropriate reagent system is selected from SO₂Cl₂; SOCl₂, PBr₃, PBr₅, and a sulfonyl halide of formula Z-SO₂-R₃, wherein R₃ is a radical selected from the group consisting of (C₁-C₄)-alkyl, phenyl, and phenyl mono- or disubstituted by a (C₁-C₄)-alkyl radical and Z is Cl or Br.

In a more preferred embodiment, the reagent system is the sulfonyl halide Z-SO₂-R₃. In a still more preferred embodiment, of the process Z-SO₂R₃ is ClSO₂Mes. When the compound (V) is reacted with the sulfonyl halide of formula Z-SO₂-R₃, a compound of formula (IV) with Y= OSO₂R₃ is obtained, which has been named (IVa).

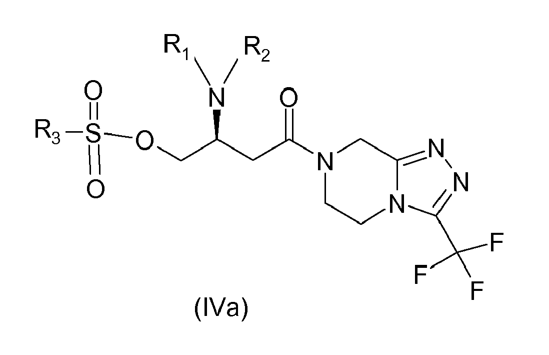

This reaction can be carried out in an appropriate solvent and in the presence of a tertiary amine, at a temperature comprised between -20 °C and room temperature. Preferably, the reaction is carried out at a temperature comprised between -10 °C and 0 °C. Common solvents for this reaction include (C₁-C₃)-chlorine-containing solvents such as methylene chloride or 1,2-dichloroethane, aromatic hydrocarbons such as toluene or xylene, ethers such as tetrahydrofuran, (C₃-C₇)-ketones such as methylethylketone, and dimethylformamide. Examples of suitable tertiary amines are diisopropylethylamine and triethylamine.

In the present invention, preferred sulfonate compounds of formula (IVa) are those where the sulfonate (IVa) is a mesylate (R₃= methyl), a besylate (R₃= phenyl) or a tosylate (R₃= 4-methylphenyl). The most preferred sulfonate (IVa) is the mesylate.

These sulfonate compounds can be used in the following step of the process as such or they can be converted into another compound of formula (IV). For instance, they can be converted into compounds of formula (IV) with Y= I by reaction with a iodine source. The compounds (IV) with Y= I has been named (IVb).

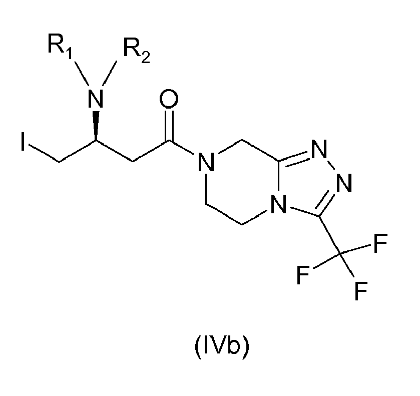

In a particular embodiment of the process, a compound (V) is reacted with a halogenating agent selected from the group consisting of SO₂Cl₂, SOCl₂, PBr₃, and PBr₅ to give a compound of formula (IV) where Y is Cl or Br. This compound has been named (IVc).

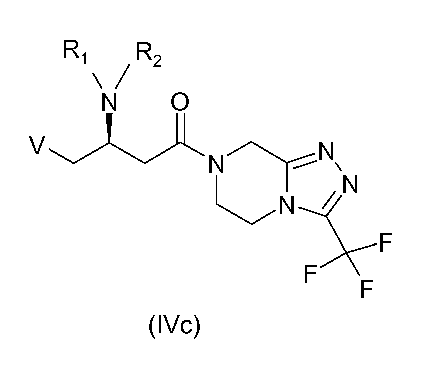

In compound (IVc), V is Cl or Br and R₁ and R₂ have the same meaning as mentioned above.

Compounds of formula (IVc) with V= Cl can also be prepared from compound (V) by reaction with CISO₃Me in the presence of a tertiary amine such as triethylamine and at a temperature comprised between about 30 °C and the reflux temperature of the solvent used. A lower temperature can be used, for instance, room temperature, increasing the reaction time until the reaction is completed.

Compounds of formula (IVc) with V = Br can be used in the following step of the process as such or they can be converted into compound of formula (IVb) by reaction with a iodine source. Compound (IVc) with V= Cl is converted into a compound of formula (IVb) by reaction with a iodine source.

Preferably, the iodine source used to carry out the displacement of the halogen or sulfonate leaving group of the compounds of formula (IVa) or (IVc) is a iodide salt such as an alkaline metal salt. Examples of appropriate alkaline metal salts include Kl, Nal, or Lil. Other appropriate iodine source is, for instance, tetrabutylammonium iodide (TBAI) or N-iodosuccinimide. The displacement can be carried out in the presence of a suitable solvent. Examples of suitable solvents are (C₃-C₅)-ethers such as tetrahydrofuran, (C₃-C₆)-ketones such as acetone or ethylmethylketone and a polar aprotic solvent such as N,N-dimethylformamide.

Compounds of formula (IVa) or (IVc) can be isolated from the reaction medium or converted into the compound (IVb) without being isolated from the reaction medium.

In another preferred embodiment of this step of the process, the appropriate reagent system is an activating agent and a iodine source, thereby first an activated derivative of the compound (V) is obtained which is then transformed into a compound of formula (IVb).

Thus, in a particular embodiment, compound (IV) is prepared by reaction of compound (V) with a reagent system selected from dicyclohexylcarbodiimide iodomethane complex, and methyltriphenoxyphosphonium iodide (C₆H₅O₃PCH₃)I. Generally, in those cases the activated intermediate derivatives are not isolated. In another particular embodiment, the conversion of compound (V) to compound (IV) is carried out using PPh₃/imidazole /I₂. In another particular embodiment, the conversion is carried out using PPh₃/N-iodosuccinimide.

The key step of the preparation process of the present invention is the coupling of a compound of formula (III) with a compound of formula (IV) to give a compound of formula (II).

This coupling can be carried out via the formation of an organocupric compound of the compound of formula (III) by reacting the compound (III) with magnesium or lithium, and then with a copper salt, followed by its reaction with compound (IV) as it is illustrated in Scheme II.

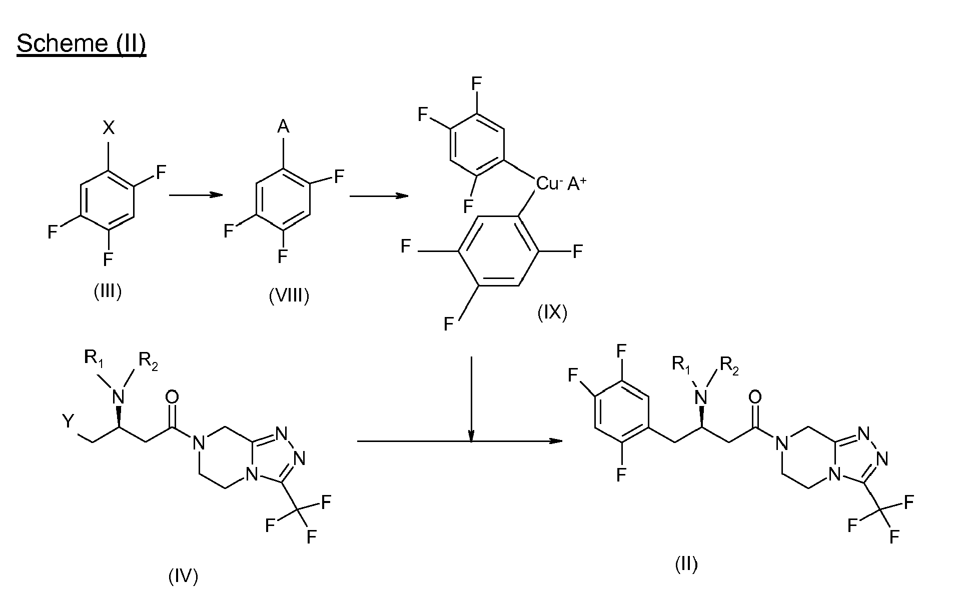

In the previous formulae R₁ is an hydrogen atom or an amino protective group; R₂ is an amino protective group; or alternatively R₁ and R₂ taken together form a phtalimido group; X is Br or I; Y is selected from Br, I, and a radical of formula R₃SO₂-, where R₃ is a radical selected from (C₁-C₄)-alkyl, phenyl, and phenyl mono- or disubstituted by a (C₁-C₄)-alkyl radical; and A is MgX or Li.

In a preferred embodiment of the preparation process, the coupling is carried out via the formation of an organocupric compound of the compound of formula (III) where X is Br.

In another preferred embodiment, compounds of formula (IV) are those where Y is I. In another preferred embodiment compounds of formula (IV) are those where R₁ is H. In another more preferred embodiment, compounds of formula (IV) are those where R₂ is benzyloxycarbonyl or t-butoxycarbonyl.

In a particular embodiment the compound (II) obtained has the following formula (IIa):

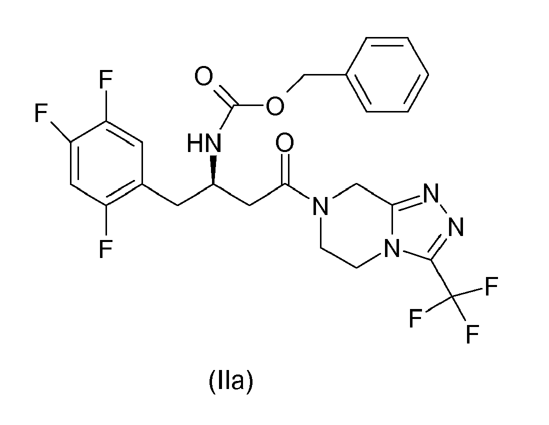

Generally, the reaction of magnesium or lithium with a compound (III) is carried out by treating the metal with a solution of the compound (III) in an appropriate solvent yielding an organomagnesium or an organolithium compound of formula (VIII). Examples of appropriate solvents are (C₂-C₈)-ethers or (C₆-C₈) hydrocarbons.

In a preferred embodiment of the process the magnesium or organolithium compound is reacted with a copper salt selected from cuprous bromide, cuprous bromide methyl sulfide complex (CuBr.SMe₂), Copper iodide (Cul), dilithium tetrachlorocuprate (Li₂CuCl₄), and dilithium dichlorocyanocuprate (Li₂CuCNCl₂). The Li₂CuCl₄ can be prepared in situ from LiCl and CuCl₂. The Li₂CuCNCl₂ can be prepared in situ from LiCl and CuCN. In a more preferred embodiment the copper salt is selected from cuprous bromide methyl sulfide complex and copper iodide.

As mentioned above compound (II) as defined above can also be obtained, first via the formation of a organozinc compound of the compound of formula (IV) as defined above, and then carrying out the coupling with compound (III) in the presence of a palladium catalyst. This coupling is known as Negishi coupling.

In a preferred embodiment of the process the organozinc compound (X) can be prepared by reaction of compound (IV) with zinc as seen in Scheme III. Organozinc compound (X) then is reacted with a compound of formula (III) in the presence of a palladium catalyst. Generally, the catalyst is generated in situ from a source of palladium such as Pd₂(dibenzylideneacetone)₃ and a phosphine such as tri-*o*-tolylphosphine in a polar aprotic solvent such as N,N-dimethylformamide or N,N-dimethylacetamide. Other examples of appropriate palladium catalyst are the following: PdCl₂(PPh₃)₂ , Pd(PPh₃)₄, Pd/C, Pd(OAc)₂, or PdCl₂(dppf) where dppf is 1,1'-bis(diphenylphosphino) ferrocene.

In a more preferred embodiment of the preparation process via Negishi reaction, compounds of formula (III) are those where X is I. In another preferred embodiment of the preparation process via Negishi, compounds of formula (IV) are those where Y is I. In another preferred embodiment compounds of formula (IV) are those where R₁ is H. In another more preferred embodiment, compounds of formula (IV) are those where R₂ is benzyloxycarbonyl or t-butoxycarbonyl.

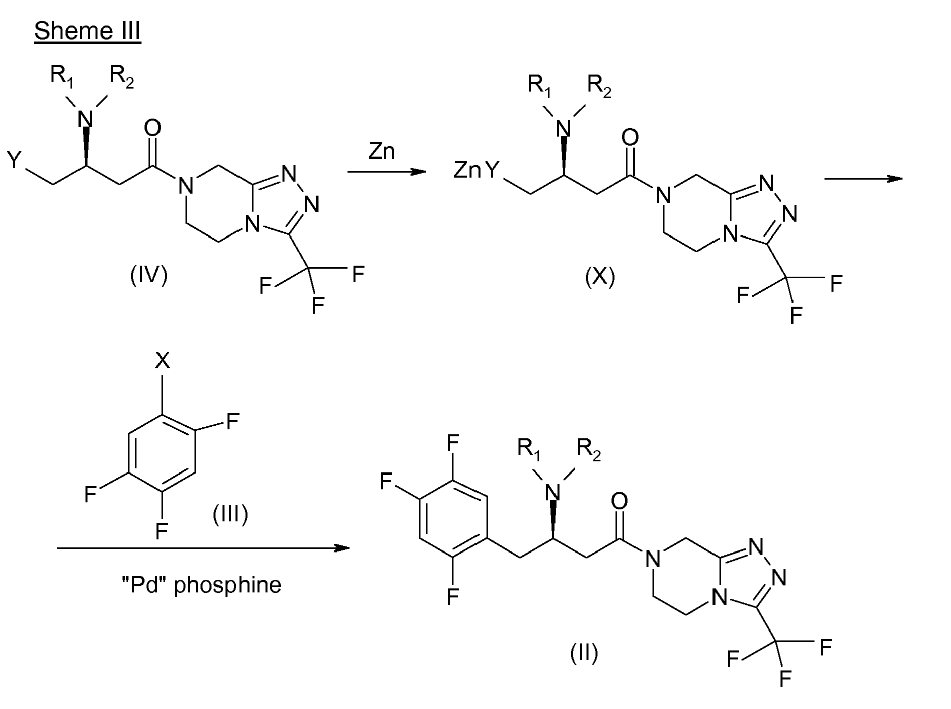

The process for the preparation of sitagliptin includes a deprotection reaction of the compound (II) as defined above to remove the protective group.

The protective group of the primary amino moiety can be removed by procedures known in the art (cf. Protective Groups in Organic Synthesis, Wiley-Interscience, (1999)). The conditions for carrying out the deprotection reaction depend on the protective group used. For instance, when a t-butoxycarbonyl (BOC) is used, the deprotection reaction can be carried out with a mixture of trifluoroacetic acid and methylene chloride or using hydrogenolitic conditions in the presence of a metal catalyst in an organic solvent. The deprotection reaction of phthalimide compounds can be carried out with hydrazine or using basic conditions. When benzyloxycarbonyl (Cbz) is used, the deprotection reaction can be carried out using hydrogenolytic conditions in the presence of a metal catalyst in an organic solvent.

The sitagliptin obtained by the process of the present invention may be converted into pharmaceutically acceptable salts by known methods described in the art, for instance by reaction of sitagliptin free base with a sufficient amount of a pharmaceutically acceptable acid to give the corresponding salt. Generally, the conversion is carried out in a suitable organic solvent such a (C₁-C₅)-alcohol, (C₁-C₅)-alkyl acetate, or water or mixtures thereof, preferably in an aqueous (C₁-C₅)-alcohol solution. Generally, the process is carried out at a temperature comprised between about 0 °C to about 100 °C. The salt can be crystallized by cooling the mixture. The salts can be isolated by filtration and drying.

The term "pharmaceutically acceptable salts" used herein encompasses any salt formed from pharmaceutically acceptable non-toxic acids including inorganic or organic acids. Example of acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methansulfonic, mucic, nitric, pamoic, pantothenic, phosphoric, succinic, sulphuric, tartaric, p-toluensulfonic acid, 10-camphorsulfonic, tartaric, dodecylsulfate and the like. Particularly preferred are citric, hydrobromic, hydrochloric, maleic, phosphoric, sulphuric, fumaric and tartaric acids. In a more preferred embodiment the pharmaceutically acceptable salt is the monobasic dihydrogen phosphate salt. The pharmaceutically acceptable salts can also be in form of hydrates. There is no limitation regarding the salts, except that if used for therapeutic purposes, they must be pharmaceutically acceptable.

The process of the present invention can be carried out in sequential steps isolating the intermediates obtained, or alternatively, some of the steps of the present invention may be carried out in one pot, as illustrated in the examples.

In a particular embodiment, the sulfonate intermediate of formula (IVa) is not isolated from the reaction medium and the subsequent iodo intermediate of formula (IVb) is then isolated from the reaction medium.

In another particular embodiment the sulfonate intermediate of formula (IVa) is isolated but not the subsequent iodo intermediate of formula (IVb) which is transformed into N-protected sitagliptin without being isolated from the reaction medium.

Intermediate compounds of formula (IV) and (V) mentioned above are new. Thus, the provision of the new compounds of formulae (IV) and (V), which are useful intermediates for the preparation of sitagliptin, also forms part of the present invention. Preferred compounds of formula (IV) and of formula (V) are those where R₁ is H and R₂ is an amino protective group, preferably selected from carbamates, amides, sulfonamides, and benzylamines. More preferably, the amino protective group is selected from *t*-butoxycarbonyl (BOC), 9-fluorenylmethoxycarbonyl (Fmoc), benzyloxycarbonyl (Cbz), and N-benzylamine. More preferred compounds of formula (IV) and of formula (V) are those where R₁ is hydrogen and R₂ is benzyloxycarbonyl. Also preferred compounds of formula (IV) are those where Y is I.

Throughout the description and claims the word "comprise" and variations of the word, are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1: Preparation of (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate

A 2.0 M solution of AlMe₃ in hexanes (0.98 mL, 1.96 mmol) was added to a solution of 3-(trifluoromethyl)-5,6,7,8-tetrahydro-[1,2,4]triazolo[4,3-a]pyrazine (271 mg, 1.41 mmol) in anhydrous methylene chloride (5 mL). The resulting mixture was added dropwise to a solution of (S)-benzyl 5-oxotetrahydrofuran-3-ylcarbamate (265 mg, 1.13 mmol) in anhydrous methylene chloride (5 mL) at 0 °C. When the reaction was completed, 1 M KHSO₄ solution (25 mL) was added slowly. The layers were separated and the aqueous layer was extracted with methylene chloride (25 mL). The organic layers were mixed, dried over anhydrous Na₂SO₄, filtered and concentrated to dryness to yield (S)-benzyl 1-hydroxy-4-oxo-4-(3-(trifluoromethyl)-5,6-dihydro-[1,2,4]triazolo[4,3-a] pyrazin-7(8H)-yl)butan-2-ylcarbamate (353 mg, 0.83 mmol, 73% yield).

### Example 2: Preparation of (S)-benzyl1-hydroxy-4-oxo-4-(3-(trifluoromethyl)-5,6-dihydro-[1,2,4]triazolo[4,3-a]pyrazin-7(8H)-yl)butan-2-ylcarbamate without solvent

(S)-Benzyl 5-oxotetrahydrofuran-3-ylcarbamate (468 mg, 1.99 mmol) and 3-(trifluoromethyl)-5,6,7,8-tetrahydro-[1,2,4]triazolo[4,3-a]pyrazine (382 mg, 1.99 mmol) were mixed and warmed up to 110 °C. The resulting mixture was allowed to stir for 12 hours. After this period of time, the crude was diluted in 40 mL of methylene chloride and treated with 25 mL of a 1 M solution of HCl. The aqueous layer was extracted with 25 mL of methylene chloride. The organic layers were mixed and washed with a saturated solution of NaHCO₃, dried over anhydrous Na₂SO₄, filtered and concentrated to dryness. The resulting crude was purified by flash column chromatography through silicagel, yielding (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate (204 mg, 0.48 mmol, 24% yield).

### Example 3: Preparation of (S)-benzyl 1-hydrox-4-oxo-4-(3-(trifluoromethyl)-5,6-dihydro-[1,2,4]triazolor[4,3-a]pyrazin-7(8H)-yl)butan-2-ylcarbamate using N-methylmorpholine as solvent

To a solution of 3-(trifluoromethyl)-5,6,7,8-tetrahydro-[1,2,4]triazolo[4,3-a]pyrazine (3.06 g, 15.9 mmol) in N-methylmorpholine (0.6 mL, 5.31 mmol), (S)-benzyl 5-oxotetrahydrofuran-3-ylcarbamate (1.25 mg, 5.31 mmol) was added. The resulting mixture was stirred at 100 °C for 20 hours. After this period of time, the reaction was concentrated to dryness. The product was purified by flash column chromatography using AcOEt:MeOH mixtures as eluent, yielding (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate (1.19 g, 2.79 mmol, 52% yield).

### Example 4: Preparation of (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate using AlCl₃/NEt₃

Triethylamine (0.36 mL, 3.56 mmol) was added to an aluminium chloride (243 mg, 1.82 mmol) suspension in 3 mL of anhydrous methylene chloride under atmosphere of N₂. The mixture was allowed to warm to room temperature. Then, a solution of 3-(trifluoromethyl)-5,6,7,8-tetrahydro-[1,2,4]triazolo[4,3-a]pyrazine (175 mg, 0.91 mmol) and (S)-benzyl 5-oxotetrahydrofuran-3-ylcarbamate (195 mg, 0.83 mmol) in 3 mL of methylene chloride was added. The mixture was allowed to react at room temperature for 24 hours. Then, the reaction was quenched with water and Na₂CO₃. The layers were separated and the organic phase was dried over anhydrous Na₂SO₄, filtered and concentrated. The resulting crude was purified by chromatography through silicagel to yield (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate (78 mg, 0.18 mmol, 22% yield).

### Example 5: Preparation of (S)-benzyl 1-iodo-4-oxo-4-(3-(trifluoromethyl)-5,6-dihydro-[1,2,4]triazolo[4,3-a]pyrazin-7(8H)-yl)butan-2-ylcarbamate

Triethylamine (71 mg, 70 mmol) was slowly added to a solution of 1-hydroxy-4-oxo-4-(3-(trifluoromethyl)-5,6-dihydro-[1,2,4]triazolo[4,3-a]pyrazin-7(8H)-yl)butan-2-ylcarbamate (251 mg, 59 mmol) in anhydrous THF (4 mL) at -10 °C. Mesyl chloride (74 mg, 65 mmol) was added dropwise and the reaction was warmed to 0 °C. When all the starting material was consumed, lithium iodide (157 mg, 1.18 mmol) was added and the resulting mixture was allowed to warm to room temperature. When the reaction was completed, water (25 mL) and methylene chloride (25 mL) were added. The organic layer was washed with 1 M Na₂S₂O₃ solution (25 mL) and brine, dried over anhydrous Na₂SO₄ and concentrated to yield (S)-benzyl 1-iodo-4-oxo-4-(3-(trifluoromethyl)-5,6-dihydro-[1,2,4]triazolo[4,3-a]pyrazin-7(8H)-yl)butan-2-ylcarbamate (204 mg, 0.38 mmol, 65% yield).

### Example 6: Preparation of 1-iodo-4-oxo-4-(3-(trifluoromethyl)-5,6-dihydro-[1,2,4]triazolo[4,3-a]pyrazin-7(8H)-yl)butan-2-ylcarbamate using PPh₃/Imidazole/I₂

Imidazole (135 mg, 1.98 mmol) and iodine (503 mg, 1.98 mmol) were added to a solution of triphenylphosphine (520 mg, 1.98 mmol) in 8 mL of methylene chloride at 0 °C. A solution of (S)-benzyl 1-hydroxy-4-oxo-4-(3-(trifluoromethyl)-5,6-dihydro-[1,2,4]triazolo[4,3-a]pyrazin-7(8H)-yl)butan-2-ylcarbamate (807 mg, 1.88 mmol) in 3 mL of methylene chloride was added dropwise. When the reaction was concluded, the mixture was filtered and concentrated to dryness. The crude was purified by column chromatography through silicagel yielding (S)-benzyl 1-iodo-4-oxo-4-(3-(trifluoromethyl)-5,6-dihydro-[1,2,4]triazolo[4,3-a]pyrazin-7(8H)-yl)butan-2-ylcarbamate (506 mg, 0.94 mmol, 50% yield).

### Example 7: Preparation of(R)-benzyl 4-oxo-4-(3-(trifluoromethyl)-5,6-dihydro-[1,2,4]triazolo[4,3-a]pyrazin-7(8H)-yl)-1-(2,4,5-trifluorophenyl)butan-2-ylcarbamate

A solution of 1-bromo-2,4,5-trifluorobenzene (638 mg, 3.02 mmol) in THF (5 mL) was slowly added to a suspension of magnesium turnings (73 mg, 3.02 mmol) in THF (5 mL). The mixture was allowed to stir for 1 hour at 50 °C. To a solution of (S)-benzyl 1-iodo-4-oxo-4-(3-(trifluoromethyl)-5,6-dihydro-[1,2,4]triazolo[4,3-a]pyrazin-7(8H)-yl)butan-2-ylcarbamate (406 mg, 0.76 mmol) in THF (5mL) at -20 °C, cuprous bromide methylsulfide complex (311 mg, 1.51 mmol) was added. The organomagnesium bromide solution was added dropwise and the mixture was allowed to warm up to room temperature. Once de reaction was completed, NH₄Cl saturated solution (20 mL) and AcOEt (20 mL) were added sequentially. The organic layer was dried with anhydrous Na₂SO₄, filtered and concentrated. Digestion in toluene:heptane (5:1) leads to the precipitation of (R)-benzyl 4-oxo-4-(3-(trifluoromethyl)-5,6-dihydro-[1,2,4]triazolo[4,3-a]pyrazin-7(8H)-yl)-1-(2,4,5-trifluorophenyl)butan-2-ylcarbamate (221 mg, 54% yield).

### Example 8: Preparation of (S)-2-(benzyloxycarbonylamino)-4-oxo-4-(3-(trifluoromethyl)-5,6-dihydro-[1,2,4] triazolo[4,3-a]pyrazin-7(8H)-yl)butyl methanesulfonate (mesyl-derivative)

Triethylamine (221 µL, 1.59 mmol) was added dropwise to a solution of (S)-benzyl 1-hydroxy-4-oxo-4-(3-(trifluoromethyl)-5,6-dihydro-[1,2,4]triazolo[4,3-a]pyrazin-7(8H)-yl)butan-2-yl carbamate (521 mg, 1.22 mmol) in 10 mL of methylene chloride at 0 °C. Then, mesyl chloride (113 µL, 1.46 mL) was added. When the reaction was concluded, the crude was diluted with 25 mL of methylene chloride and 25 mL of water were added. The organic layer was washed with brine, dried over anhydrous Na₂SO₄ filtered and concentrated to yield (S)-2-(benzyloxycarbonylamino)-4-oxo-4-(3-(trifluoromethyl)-5,6-dihydro-[1,2,4]triazolo[4,3-a]pyrazin-7(8H)-yl)butyl methanesulfonate (555 mg, 1.10 mmol, 90% yield).

### Example 9: Preparation of (R)-benzyl 4-oxo-4-(3-(trifluoromethyl)-5,6-dihydro-[1,2,4]thazolo[4,3-a]pyrazin-7(8H)-yl)-1-(2,4,5-thfluoro phenyl)butan-2-ylcarbamate (Cbz-sitagliptin) from the isolated mesyl derivative

Sodium iodide (147 mg, 0.98 mmol) was added to a solution of (S)-2-(benzyloxycarbonylamino)-4-oxo-4-(3-(trifluoromethyl)-5,6-dihydro-[1,2,4]triazolo[4,3-a]pyrazin-7(8H)-yl)butyl methanesulfonate (247 mg, 0.49 mmol) in 2 mL of anhydrous THF at room temperature. This solution was allowed to react for 12 hours and then cooled down to -20 °C and cuprous bromide methylsulfide complex (301 mg, 1.47 mmol) was added. A solution of 1-bromo-2,4,5-trifluorobenzene (619 mg, 2.93 mmol) in 5 mL of THF was slowly added to a suspension of magnesium turnings (70 mg, 2.93 mmol) in 5 mL of THF. The mixture was allowed to stir for 1 hour at 50 °C. After this period of time, this organomagnesium bromide solution was added dropwise to the reaction and the mixture was allowed to warm up to room temperature. Once de reaction was completed, NH₄Cl saturated solution (20 mL) and AcOEt (20 mL) were added sequentially. The organic layer was dried with anhydrous Na₂SO₄, filtered and concentrated. Apolar impurities were washed with heptane. The resulting oil was diluted in AcOEt, treated with charcoal, filtered and concentrated. Digestion in toluene:heptane (5:1) leads to the precipitation of (R)-benzyl 4-oxo-4-(3-(trifluoromethyl)-5,6-dihydro-[1,2,4]triazolo[4,3-a]pyrazin-7(8H)-yl)-1-(2,4,5-trifluorophenyl)butan-2-ylcarbamate (112 mg, 40% yield).

### Example 10: Preparation of (R)-3-Amino-1-(3-(trifluoromethyl)-5,6-dihydro-[1,2,4]triazolo[4,3-a]pyrazin-7(8H)-yl)-4-(2,4,5-trifluorophenyl)butan-1-one

(R)-Benzyl 4-oxo-4-(3-(trifluoromethyl)-5,6-dihydro-[1,2,4]triazolo[4,3-a]pyrazin-7(8H)-yl)-1-(2,4,5-trifluorophenyl)butan-2-ylcarbamate (203 mg, 0.37 mmol) was dissolved in methanol (15 mL), treated with 10 mol % Pd/C (20 mg) and stirred under a hydrogen atmosphere until reaction completion. The mixture was filtered through celite and the organic solvent was evaporated to dryness to give (R)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one (151 mg, 99% yield).

## Claims

1. A process for the preparation of sitagliptin of formula (I), or a pharmaceutically acceptable salt thereof, or a solvate thereof, including a hydrate, 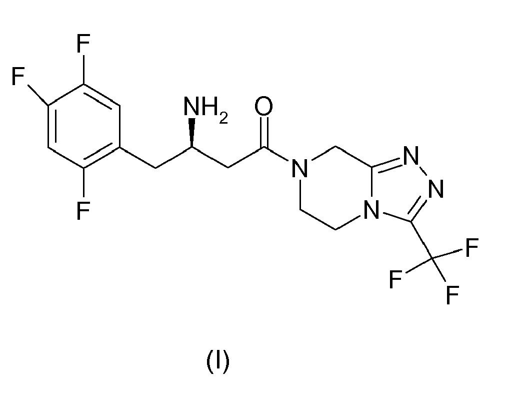 which comprises the steps of:
a) coupling a compound of formula (III) with a compound of formula (IV) to give a compound of formula (II); 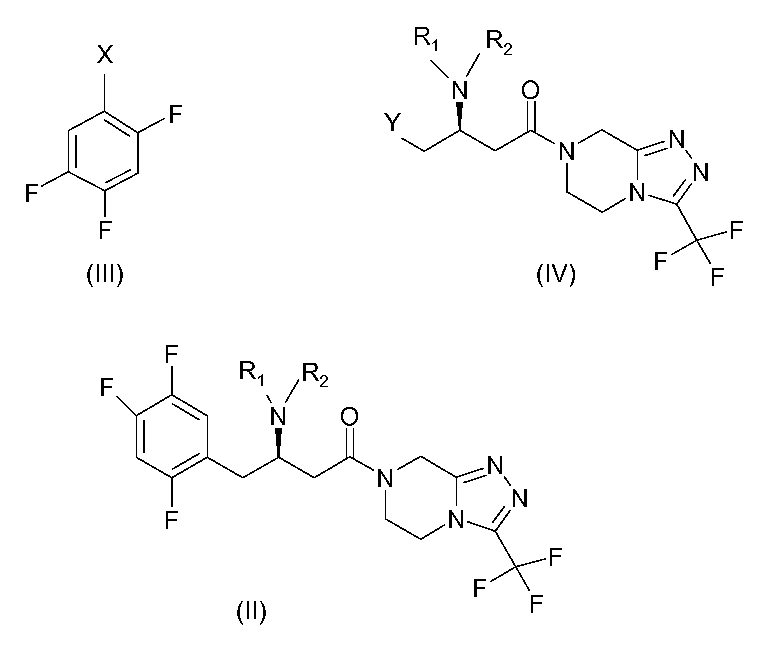 the coupling being carried out first via the formation of an organocupric compound of the compound of formula (III) by reacting the compound (III) with magnesium or lithium to give an organomagnesium or an organolithium compound, and then with a copper salt to give an organocupric compound or, alternatively, first via the formation of a organozinc compound of the compound of formula (IV) by reacting compound (IV) with zinc, and then carrying out the coupling of the organozinc compound thus obtained with the compound of formula (III) in the presence of a palladium catalyst;
wherein:
R₁ is an hydrogen atom or an amino protective group; R₂ is an amino protective group; or alternatively R₁ and R₂ taken together form a phtalimido group;
X is Br or I; and
Y is selected from the group consisting of Br, I and a radical of formula R₃SO₂- wherein R₃ is a radical selected from the group consisting of (C₁-C₄)-alkyl, phenyl, and phenyl mono- or disubstituted by a (C₁-C₄)-alkyl radical;
b) submitting the compound (II) obtained in step a) to a deprotection reaction to remove the protective groups to yield sitagliptin (I) as free base; and
c) optionally, treating the compound obtained in step b) with a pharmaceutically acceptable acid to form the corresponding pharmaceutically acceptable salt.

2. The preparation process according to claim 1, wherein R₁ is hydrogen.

3. The preparation process according to claim 2, wherein the amino protective group R₂ is *t*-butoxycarbonyl or benzyloxycarbonyl.

4. The preparation process according to any of the claims 1-3, wherein the coupling is carried out via the formation of an organocupric compound of the compound of formula (III) wherein X is Br.

5. The preparation process according to any of the claims 1-4 wherein Y is 1.

6. The preparation process according to any of the claims 1-5, wherein step
a) is carried out with magnesium and the copper salt is selected from the group consisting of cuprous bromide, cuprous bromide methyl sulfide complex, copper iodide, dilithium tetrachlorocuprate and dilithium dichlorocyanocuprate.

7. The preparation process according to any of the claims 1-6, further comprising a previous step of reacting a compound of formula (V) with an appropriate reagent system to convert the OH functional group into a leaving group Y, yielding the compound of formula (IV); and thereafter as necessary transforming a compound of formula (IV) into another compound of formula (IV), wherein R₁, R₂, and Y have the same meaning as mentioned in claim 1, Y further including Cl.

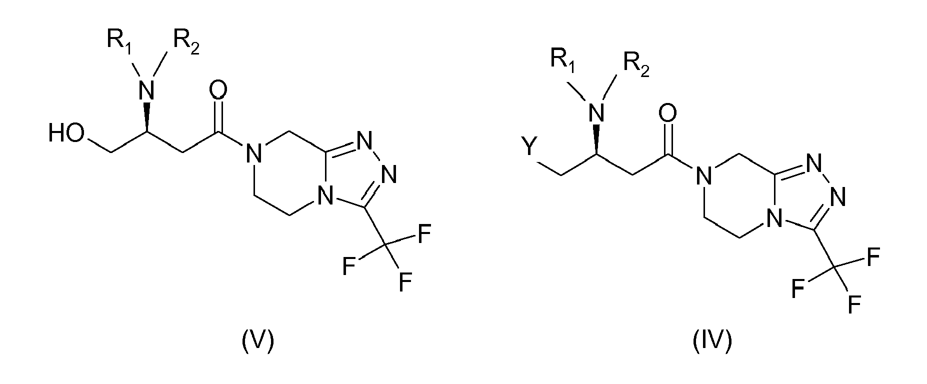

8. The preparation process according to claim 7, wherein the appropriate reagent system is selected from the group consisting of SO₂Cl₂, SOCl₂, PBr₃, PBr₅ and a sulfonyl halide of formula Z-SO₂-R₃, wherein R₃ is a radical selected from the group consisting of (C₁-C₄)-alkyl, phenyl, and phenyl mono- or disubstituted by a (C₁-C₄)-alkyl radical and Z is Cl or Br.

9. The preparation process according to claim 8, wherein the sulfonyl halide Z-SO₂-R₃ is ClSO₂Me.

10. The preparation process according to any of the claims 7-9 wherein a compound of formula (IV) wherein R₁ and R₂ are as mentioned in claim 1 and Y is Cl, Br or a radical of formula R₃SO₂- wherein R₃ is a radical selected from the group consisting of (C₁-C₄)-alkyl, phenyl, and phenyl mono- or disubstituted by a (C₁-C₄)-alkyl radical, is transformed into another compound of formula (IV) where Y is I, by reaction with a iodine source.

11. The preparation process according to claim 10, wherein the iodine source is selected from the group consisting of a metal alkaline iodide salt, tetrabutylammonium iodide, and N-iodosuccinimide.

12. The preparation process according to claim 7, wherein the appropriate reagent system is an activating agent and a iodide source, thereby first an activated derivative of the compound (V) is obtained which is transformed into a compound of formula (IV) with Y= I having the formula (IVb).

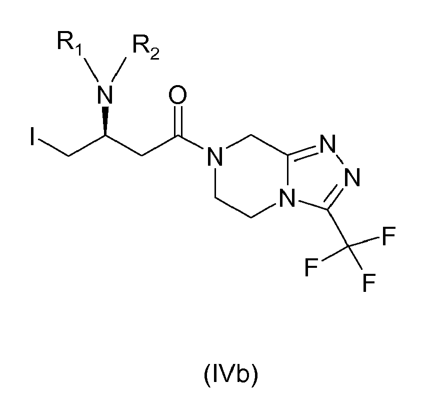

13. The preparation process according to claim 12, wherein the appropriate reagent system is selected from the group consisting of dicyclohexylcarbodiimide iodomethane complex, methyltriphenoxyphosphonium iodide, PPh₃/imidazole /I₂, and PPh₃/N-iodosuccinimide.

14. The preparation process according to any of the claims 7-13, further comprising a previous step of reacting a compound of formula (VI) with a compound of formula (VII) to give a compound of compound (V); 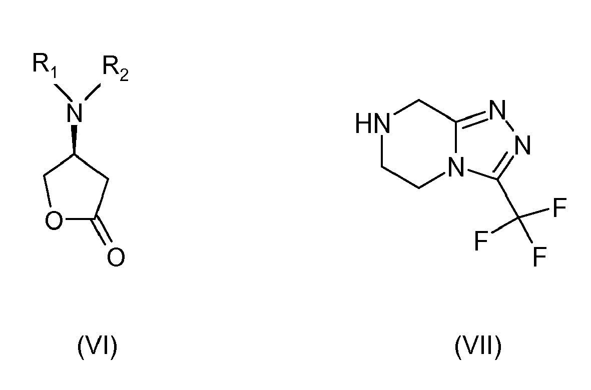 where R₁ is an hydrogen atom or an amino protective group; R₂ is an amino protective group; or, alternatively, R₁ and R₂ taken together form a phtalimido group, and compound (VII).

15. A compound of formula (IV): 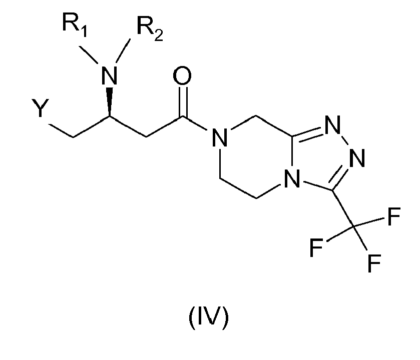 wherein:
R₁ is an hydrogen atom or an amino protective group; R₂ is an amino protective group; or alternatively R₁ and R₂ taken together form a phtalimido group; and
Y is selected from the group consisting of Cl, Br; I and a radical of formula R₃SO₂-wherein R₃ is a radical selected from the group consisting of (C₁-C₄)-alkyl, phenyl, and phenyl mono- or disubstituted by a (C₁-C₄)-alkyl radical;
with the proviso that when R₁ is H and R₂ is t-butoxycarbonyl, then R₃ is not methyl.

16. A compound of formula (V): 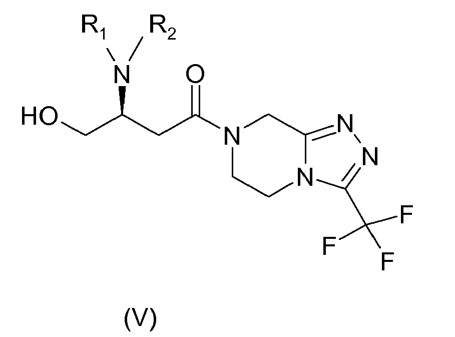 wherein:
R₁ is an hydrogen atom or an amino protective group; R₂ is an amino protective group; or alternatively R₁ and R₂ taken together form a phtalimido group;
with the proviso that when R₁ is H then R₂ is not t-butoxycarbonyl.
